# EUROPEAN PATENT APPLICATION

(11) **EP 4 652 944 A1**
(43) Date of publication of application: **26.11.2025**
(21) Application number: 25178165.4
(22) Date of filing: 22.05.2025
(51) Int. Cl.: A61B 17/34

(54) **SYSTEM AND METHOD TO GUIDE A NEEDLE**

(30) Priority: 24.05.2024 US 202418673692
(71) Applicant: Varian Medical Systems, Inc., Palo Alto, CA 94304 (US)
(72) Inventor: GUERIN, Erwan, 68300 Saint-Louis (FR)
(74) Representative: Mathisen & Macara LLP

(57) **Abstract**

A needle guide device includes a needle 240; a controller 202; and a display 204 driven by the controller and configured to indicate a difference between an ideal angle and a current angle of the needle.

## Description

### BACKGROUND

The present disclosure relates to needles, such as surgical needles. More specifically, the present disclosure relates to a system and method to guide a needle to a target position, e.g. to guide a needle during surgery to a target position.

Needles are used during different non-surgical and surgical procedures, e.g. performing injection, biopsy, cryogenic ablation of tissue, and microwave ablation. Conventionally, the insertion of a needle is performed after imaging or scanning a patient to understand the current state of the patient's body and location of a target area or target tissue. With this imaging scan as part of pretreatment planning, an entry point of the needle on the patient's body, a target position of the needle, and an angle and a distance between the entry point and the target position can be determined.

During a conventional procedure, a clinician localizes the needle entry point and inserts a small portion of the needle in the body of the patient. During needle insertion, the clinician scans again to determine the needle's position with respect to the target area, makes any adjustments, and continues to insert the needle inside the patient. Step by step, after several iterations of scanning and repositioning, the needle can be located at the target position. When the needle is at the expected depth and angle, the clinician performs a task or treatment. When the task or treatment is finished, the needle is removed from the patient.

Insertion and location of the needle to the target position in this manner is cumbersome, slow, and inexact.

### SUMMARY

By tracking the specific angle of the needle (e.g. with accelerometer, gyroscope, and/or distance sensor data), the disclosed system can provide different technical features including (i) determining the ideal angle of the needle, (ii) measuring the current angle of the needle, (iii) calculating the difference between the ideal angle and the current angle of the needle, (iv) determining an insertion depth of the needle, (v) measuring the current depth of the needle, and/or (vi) calculating the difference between the insertion depth and the current depth of the needle.

As a result, a user or clinician may not need to scan the patient multiple times while inserting the needle. Thus, the patient may receive less radiation during the procedure, the procedure may take less time, the clinician may realize more precision for locating the needle for treatment, the patient may experience less discomfort, the clinician may visualize directly on the needle guide the direct information and feedback for locating the needle, and more procedures could be performed in one day by the medical facility.

According to an embodiment, a needle guide device for a needle includes a controller; and a display driven by the controller and configured to indicate a difference between an ideal angle and a current angle of the needle.

In an aspect, the needle guide device includes the needle.

In an aspect, the needle is replaceable.

In an aspect, the display indicates the current angle.

In an aspect, the current angle is based on data from a gyroscope of the needle guide device.

In an aspect, the difference between the ideal angle and the current angle is based on data from a gyroscope and an accelerometer of the needle guide device.

In an aspect, the display is further configured to indicate a distance between a current position of the needle and a target position.

The needle device can further include a distance measurer to determine a distance between a current position of the needle and a target position.

In an aspect, the distance between the current position of the needle and the target position is based on data from a distance sensor of the needle guide device.

In an aspect, the display indicates the current angle as a reticle and the ideal angle as an icon.

According to another embodiment, a method of guiding a needle includes determining an ideal angle from an entry point of a needle of needle guide in a patient to a target position of the needle; and displaying an icon on the needle guide that indicates a difference between a current angle of the needle guide and the ideal angle.

The needle may be for treatment of the patient.

The method can further include determining a difference between an ideal depth to the target position and the current depth of the needle.

In an aspect, the icon indicates a difference between a current depth of the needle and an ideal depth of the needle to the target position.

In an aspect, the current angle is determined based on data from a gyroscope and an accelerometer of the needle guide.

In an aspect, the current angle is displayed as a reticle.

In an aspect, the icon changes size to indicate the difference between the current depth and the ideal depth.

According to another embodiment a needle guide system includes the needle guide; and a computer in communication with the needle guide and capable of determining the ideal angle and transmitting the ideal angle to the needle guide.

In an aspect, the computer is further capable of determining an ideal depth of a needle of the needle guide between an entry point of a needle of the needle guide in a patient to a target position of the needle for treatment of the patient.

The system can further include a distance measurer to determine a distance between a current position of the needle and a target position of the needle for treatment of the patient.

According to another embodiment, a non-transitory computer-readable medium includes executable instructions that when executed by a processor cause the processor to perform the steps of: determining a difference between an ideal angle that is an angle from an entry point of a needle of a needle guide in a patient to a target position of the needle for treatment of the patient; and driving a display to display an icon on the needle guide that indicates a difference between a current angle of the needle guide and the ideal angle.

In an aspect, the non-transitory computer-readable medium can further cause the processor to drive the display to display the icon to indicate a difference between a current depth of the needle in the patient and an ideal depth of the needle to the target position.

In an aspect, the non-transitory computer-readable medium further cause the processor to perform the method of guiding a needle mentioned above.

The above and other features, elements, characteristics, steps, and advantages of the present invention will become more apparent from the following detailed description of preferred embodiments of the present invention with reference to the attached drawings.

### BRIEF DESCRIPTION OF THE DRAWINGS

FIG. 1 is a system block diagram showing that a position of a needle can be determined by angle and distance.
FIG. 2 is a block diagram of a needle guide system according to an exemplary embodiment.
FIG. 3 is a diagram illustrating an application of the needle guide system.
FIG. 4 shows two examples of how needle alignment can be visually indicated to a clinician while using the needle guide system.
FIG. 5A and FIG. 5B show conditions of a circular icon indicating needle position.
FIG. 6 illustrates that a coordinate system can be used to determine a difference between an ideal angle and a current angle of the needle guide system.
FIG. 7 is a flowchart of a method of guiding a needle according to an exemplary embodiment.

### DETAILED DESCRIPTION

Reference will now be made in detail to embodiments of the present disclosure, examples of which are illustrated in the accompanying drawings. Whenever possible, the same reference numerals will be used throughout the drawings to refer to the same or like features. However, this disclosure may be embodied in many different forms and should not be construed as limited to the embodiments set forth herein.

Directional terms as used herein - for example up, down, right, left, front, back, top, bottom, vertical, horizontal - are made only with reference to the figures as drawn and are not intended to imply absolute orientation.

Unless otherwise expressly stated, it is in no way intended that any method set forth herein be construed as requiring that its steps be performed in a specific order, nor that with any apparatus, specific orientations be required. Accordingly, where a method claim does not actually recite an order to be followed by its steps, or that any apparatus claim does not actually recite an order or orientation to individual components, or it is not otherwise specifically stated in the claims or description that the steps are to be limited to a specific order, or that a specific order or orientation to components of an apparatus is not recited, it is in no way intended that an order or orientation be inferred, in any respect. This holds for any possible non-express basis for interpretation, including: matters of logic with respect to arrangement of steps, operational flow, order of components, or orientation of components; plain meaning derived from grammatical organization or punctuation, and; the number or type of embodiments described in the specification.

As used herein, the singular forms "a," "an," and "the" include plural references unless the context clearly dictates otherwise. Thus, for example, reference to "a" component includes aspects having two or more such components, unless the context clearly indicates otherwise.

In the following description, reference is made to the accompanying drawings that form a part thereof, and in which is shown by way of illustrating specific exemplary embodiments in which the disclosure may be practiced. These embodiments are described in sufficient detail to enable those skilled in the art to practice the concepts disclosed herein, and it is to be understood that modifications to the various disclosed embodiments may be made, and other embodiments may be utilized, without departing from the scope of the present disclosure. The following detailed description is, therefore, not to be taken in a limiting sense.

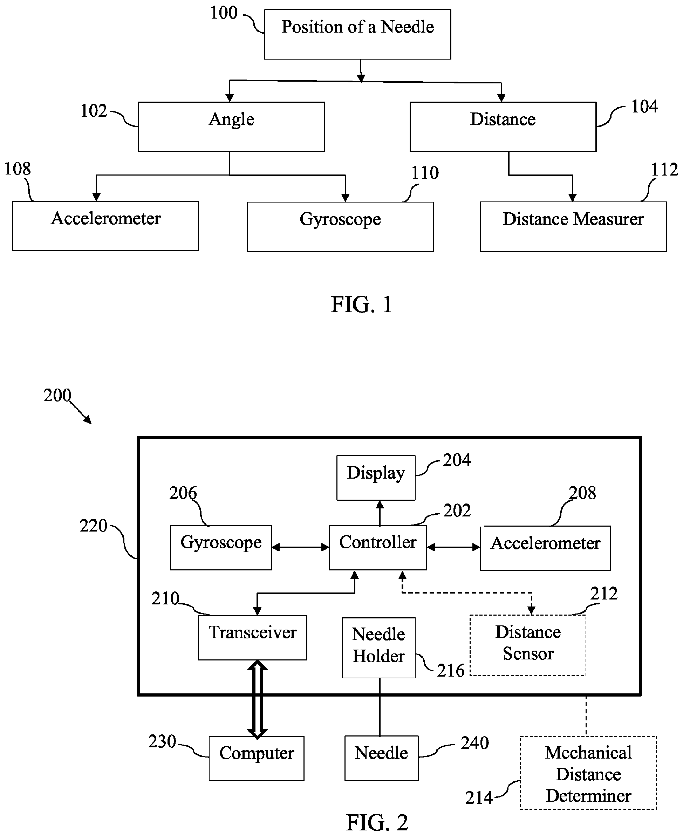

As shown in FIG. 1, a system including a needle tracks angle 102 and insertion distance 104 of the needle to assist a user in locating a position of the needle 100. Although intended for surgical application, it can be appreciated that other applications can be contemplated. For example, different components can provide data about the angle 102 and the distance 104: an accelerometer 108, a gyroscope 110, and a distance measurer 112.

FIG. 2 is a block diagram of a needle guide system 200 to guide a needle according to an exemplary embodiment. As shown the system 200 can include several components including a handheld needle guide device including a controller 202, a display 204, a gyroscope 206, an accelerometer 208, a transceiver 210 for near field communications (NFC), a distance sensor 212, a mechanical distance determiner 214, a needle holder 216 and a needle 240 coupled to the needle holder 216. These components can be assembled within a housing or enclosure 220. The system 200 can further include a computer 230 in near field communications with the controller 202 via the transceiver 210.

The controller 202 can be one or more of a processor, microcontroller, field programmable gate array (FPGA), application specific integrated circuits (ASIC), or any suitable combination of these or other components capable of executing particular sets of instructions stored in memory and capable of interfacing with components shown in FIG. 2.

The display 204 can be an electronic display that can be a liquid crystal display (LCD), an organic light-emitting display (OLED), electrophoretic display, or any other suitable display capable of showing images described below. In an embodiment, the display 204 is round.

The gyroscope 206 and accelerometer 208 can provide data used by the controller 202 and/or the computer 230 to determine the position and orientation of the needle 240 within the guide system 200.

The transceiver 210 can provide a wireless data interface to connect the controller 202 and the computer 230 via NFC. NFC can include any of WiFi, Bluetooth^{®}, Zigbee, or any other suitable technology.

The distance sensor 212 can be optional as indicated by the dashed lines. The distance sensor 212 can provide data to the controller 202 and/or computer 230 used to determine a distance from a reference location to a patient's skin to determine how deep the needle 240 has penetrated. The distance sensor 212 can include at least one configuration to provide data to determine how deep the needle 240 has penetrated. In an embodiment, the distance sensor 212 can measure a distance between the housing and the patient's skin, which would decrease as the needle 240 is being inserted. In an embodiment, the distance sensor 212 can be a pressure sensor or transducer that could indicate an increase in pressure as the needle 240 is being inserted. Other options are possible.

The mechanical distance determiner 214 can be optional as indicated by the dashed lines. It should be understood that the system 200 can include one or the other or both the distance sensor 212 and the mechanical distance determiner 214. As an alternative to the distance sensor 212, needle depth can be determined mechanically with a visual indicator such as a graduated scale or series of color coded portions on the needle 240. Alternatively, maximum needle depth can be limited by (i) a mechanical stop that physically limits the depth in which the needle 240 can be inserted or (ii) a length of the needle 240.

The needle holder 216 can be any mechanism used to attach the needle 240 to the housing 220. Such a mechanism can be magnetic or mechanical such as a bayonet or screw mount. The needle holder 216 can be capable of holding needles 240 of various sizes, lengths, and gauges necessary to suit a variety of surgical applications.

The housing 220 can be of any suitable geometric configuration capable of retaining the components with suitable interconnections and be suitable for handling during surgery. The housing 220 can be environmentally sealed and capable of being sterilized.

The computer 230 can have one or more processors and at least one memory for storing program instructions. The one or more processors can be a single microprocessor or multiple microprocessors, field programmable gate arrays (FPGAs), digital signal processors (DSPs), or any suitable combination of these or other components capable of executing particular sets of instructions. Computer-readable instructions can be stored on a tangible non-transitory computer-readable medium, such as a flexible disk, a hard disk, a CD-ROM (compact disk-read only memory), an MO (magneto-optical), a DVD-ROM (digital versatile disk-read only memory), a DVD RAM (digital versatile disk-random access memory), or a semiconductor memory. Alternatively, the methods disclosed herein can be implemented using hardware components or combinations of hardware and software such as, for example, ASICs (application specific integrated circuits), special purpose computers, or general purpose computers.

While FIG. 2 illustrates the computer 230 as a single device, in some embodiments, multiple devices (e.g., computers) may implement the functionality associated with the control server. The one or more processors may further be capable of using cloud storage as well as any future memory or storage capabilities to be implemented in the future, including, without limitation, storage capabilities that may be essential for implementing the Internet of Things (IoT).

The needle 240 can be of any suitable size, length, gauge, and configuration suitable for the surgical application. The needle 240 can be cannulated or a solid probe. The needle 240 can be separable from the needle holder 214 and disposed of following a surgery such that the remainder of the system 200 can be reusable. Optionally, the entire system 200 can be configured for single-use and disposable.

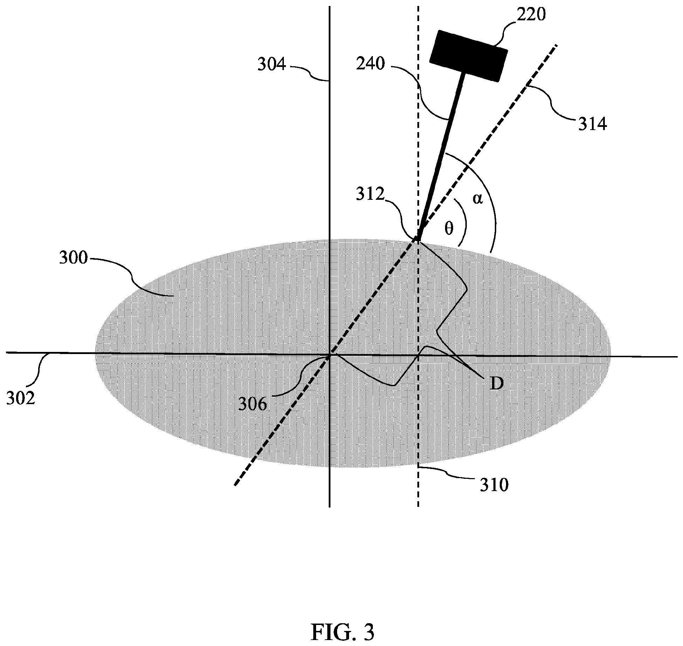

FIG. 3 is a diagram illustrating an application of the needle guide system 200. In FIG. 3, a target position 306 can been located in a patient's body 300 using scanning and/or imaging of the patient during pretreatment planning with the coordinates or location information determined or otherwise available on the computer 230 in communication with the controller 202. As shown, the target position 306 can be at an intersection of two orthogonal reference axes 302 and 304 that have been determined or provided via the pretreatment planning. The entry point 312 for the needle 240 on the patient's body 300 can also be been determined during pretreatment planning and can be along an entry point reference axis 310.

As shown in FIG. 3, the needle 240 is at an angle α with respect to a surface of the patient's body 300. An ideal axis 314 can be oriented at the ideal angle θ of the needle 240 with respect to the patient's body 300. The ideal axis 314 is predetermined as a function of the target position 306 and the entry point 312. The needle 240 should be aligned along the ideal axis 314 and inserted to the ideal depth D, a distance between the entry point 312 and the target position 306 along the ideal axis 314. When properly aligned along the ideal axis 314 and at the ideal depth D, the point of the needle 240 will be at the target position 306.

The system 200 can track the current angle of the needle α with data from at least the accelerometer 208 and gyroscope 206. The system 200 can compare the current angle α of the needle to the ideal angle θ of the needle 240 that is along the ideal axis 314 provided by the computer 230, determine a difference between the ideal angle θ and the current angle α, and provide a visual indication on the display 204 to the clinician on how to orient the needle 240 to be aligned along the ideal axis 314.

Likewise, the system 200 can track the current depth of the needle with distance sensor data for the distance sensor 212. The system 200 can compare the current depth of the needle 240 to the ideal depth D of the needle 240 that is along the ideal axis 314 provided by the computer 230 and determine a difference between the ideal depth D and the current depth and provide a visual indication on the display 204 to the clinician on how to move the needle 240 to be at the ideal depth D. The ideal depth D can be calculated as a distance between the entry point 312 and the target position 306 along the ideal axis 314.

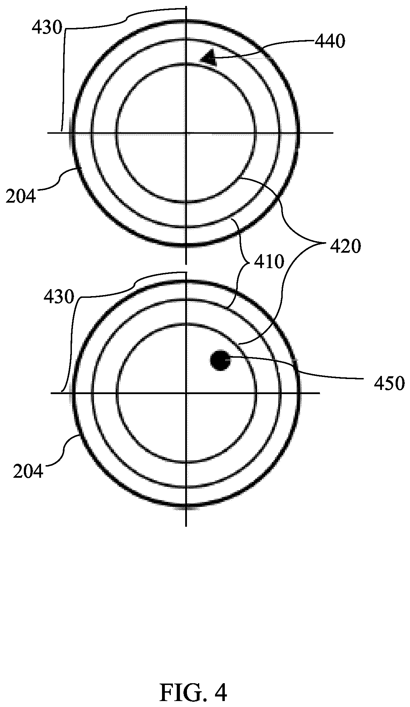

FIG. 4 shows two examples of how needle alignment can be visually indicated to a clinician while using the needle guide system 200, according to an embodiment. FIG. 4 shows that the display 204 can be round or provide a round indicator including a series of concentric circles 410 and 420. The indicator can also include a reticle or perpendicular crosshairs 430, the intersection of which can be at the center of the display 204 and indicate a position of the current angle α. The circles 410, 420, and the reticle 430 can be color coded or different colors to aid visual understanding while using the system 200.

In the representation in the upper portion of FIG. 4, a triangle icon 440 can be used to indicate coarse misalignment of the current angle α to the ideal angle θ and point in a direction the needle 240 should be moved toward the ideal angle θ. The concentric circles 410, 420 can indicate a threshold of a predetermined distance to the ideal angle θ. In the representation in the lower portion of FIG. 4, a circular icon 450 can be used to indicate fine misalignment of the current angle α to the ideal angle θ when the needle 240 is closer to the ideal angle θ and a direction in which the needle 240 should be moved toward the ideal angle θ. When the needle 240 has been moved such that the circular icon 450 is centered on the reticle 430, the needle 240 will be oriented at the ideal angle θ. In some embodiments, colors and sizes of the icons 440 and 450 and visual references of the reticle 430 and concentric circles 410, 420 can change to provide visual feedback on needle alignment conditions. For example, the size of the icons 440, 450 can become larger as the current depth of the needle approaches the ideal depth D.

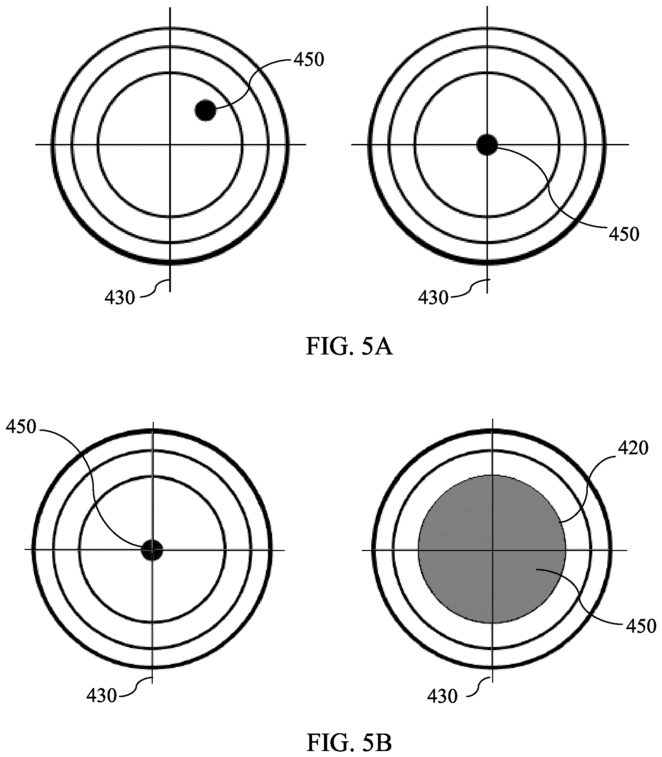

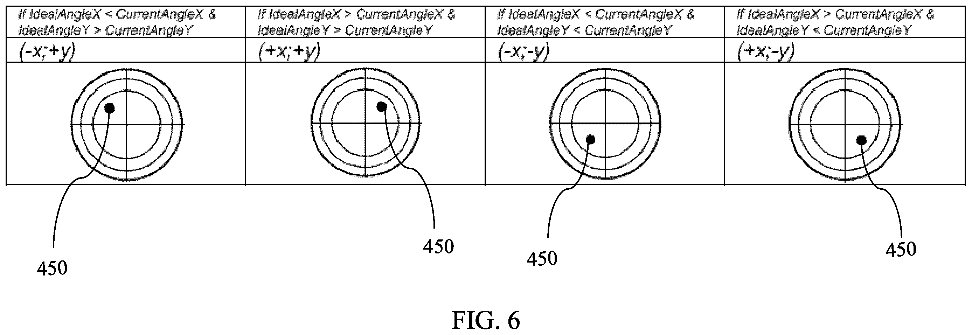

In such a way, the display 204 driven within the system 200 can indicate the current angle α, a relative direction to the ideal angle θ, a relative difference between the current angle α and the ideal angle θ, and a relative distance between the current depth and the ideal depth D. This is described in more detail with respect to FIGS. 5 and 6.

The left side of FIG. 5A shows a condition where the circular icon 450 indicates that the ideal axis 314 is aligned with a position up and to the right with respect to the current angle α, i.e. the center of the reticle 430. The right side of FIG. 5A shows a condition where the circular icon 450 indicates that the ideal axis 314 at the ideal angle θ is aligned with the current angle α. That is, the circular icon 450 is located in the center of the reticle 430.

FIG. 6 illustrates that a two-dimensional coordinate system can be used to determine a difference between the ideal angle θ and the current angle α. For example, a position of the current angle α represented at the center of the reticle 430 can be at an origin point where x = 0 and y = 0 (0,0). The ideal angle θ, represented by the position of the circular icon 450 can be at a point relatively to the center of the reticle 430. Consecutively, left to right along the examples shown in FIG. 6, the ideal angle θ is at a point where x is less than the current angle α and y is greater than the current angle α (-x,+y) in the leftmost image; the ideal angle θ is at a point where x and y are greater than the current angle α (+x,+y) in the next image; the ideal angle θ is at a point where x and y are less than the current angle α (-x,-y) in the next image; and the ideal angle θ is at a point where x is greater than the current angle α and y is less than the current angle α (+x,-y) in the rightmost image.

Although not shown, it can be understood that similarly, a depth of the needle 240 can be determined using a difference in a z direction between where the tip of the needle is calculated to be and the target position 306 along the ideal axis 314.

Like the right side of FIG. 5A, the left side of FIG. 5B shows a condition where the circular icon 450 indicates that the ideal axis 314 is aligned with the current angle α. The left side of FIG. 5B shows a condition where the circular icon 450 indicates that the ideal axis 314 is aligned with the current angle α but the depth of the needle 240 needs to be closer to the target position 306. As the needle 240 is inserted deeper into the patient toward the target position 306, the diameter of the circular icon 450 can grow relatively. For example, as shown on the right side of FIG. 5B, reaching the ideal depth D can correspond with the circular icon 450 becoming the same diameter as one of the concentric circles 420. Additionally, the color of the circular icon 450 can change once the ideal depth D is reached along the ideal axis 314, indicating that the tip of the needle 240 is at the target position 306. For example, the color of the circular icon 450 can change from red to green once the system 200 indicates that the needle 240 is at the target position 306.

In some embodiments, the circular icon 450 can blink to indicate that the tip of the needle 240 is not at the target position 306 or blink when the tip of the needle 240 is at the target position 306. In some embodiments, a changing rate of a blinking circular icon 450 can be used to indicate that the tip of the needle 240 is closer or farther away from the target position 306. For example, a faster blinking rate of the circular icon 450 can indicate that the tip of the needle 240 is father away from the target position and the blinking rate can be slowed down as the tip of the needle 240 approaches the target position 306. The circular icon 450 can stop blinking once the tip of the needle 240 is at the target position 360.

Similarly, a changing color scheme can be used to indicate relative depth of the needle 240 with respect to the target position 306. For example, a color of red can indicate that the depth of the needle 240 is farther from the target position 306, change to yellow as the tip of the needle 240 is closer to the target position 306, and change to green when the tip of the needle 240 is at the target position 306. It can be understood that different color schemes can be used, different gray shades of colors can be used, changing brightness of the circular icon 450, or any combination can be used to indicate relative position of the tip of the needle 240 to the target position 306 to aid a clinician in understanding situational awareness of where the needle 240 is and needs to be positioned for treatment.

In such a manner, the clinician can be guided to quickly and easily find the ideal angle θ along the ideal axis 314 from the entry point 312. Using the system needle guide 200, the clinician can orient the needle 240 in the proper direction and ideal depth D to reach the target position 306 as indicated directly on the system 200 by using a visual indication on the display 204.

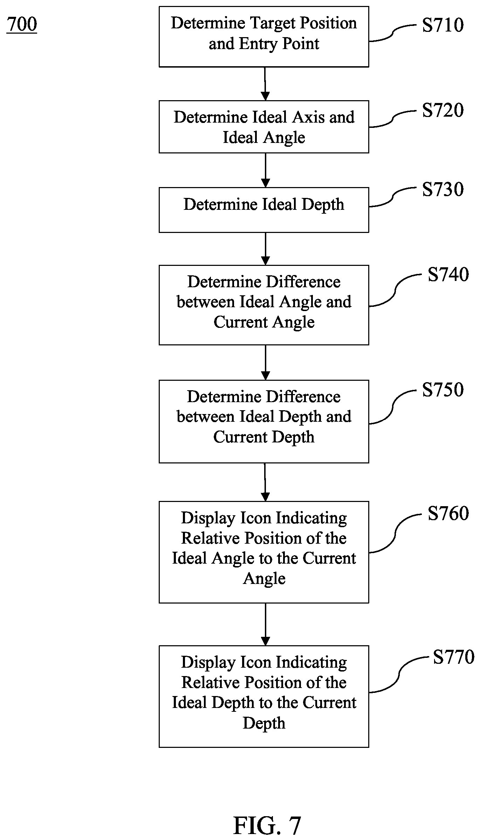

FIG. 7 is a flowchart of a method of guiding a needle 700 according to an embodiment. Initially, patient pretreatment planning is performed, conventionally using imaging and analysis to determine a target position of a needle placement within a target area and an entry point in which the needle is to be inserted in the patient, as shown in step S710. The pretreatment planning can be assisted by a computer and application program or programs that capture, format, and analyze imaging data. The application can recommend the target position and the entry point and/or be interactive such that a clinician can confirm and/or change the recommendations from the application or select the target position and the entry point based on pretreatment information and experience.

In step S720, the ideal axis can be determined as a straight line through the target position and the entry point. This determination can be computerized and made using a 3D coordinate system or via any other suitable method. Likewise, the ideal angle can be determined as an angle from the ideal axis to a reference point. For example, the reference point can be along a horizontal plane normal to the entry point or any other suitable location. The ideal angle can be determined by or input to a needle guide system.

In step 730, the ideal depth can be calculated as a difference in distance or length between the target position and the entry point along the ideal axis. The ideal depth can be determined by or input to the needle guide system.

In step 740, a difference between the ideal angle and a current angle of the needle guide system can be determined. The current angle can be calculated as an angle between a longitudinal axis of a needle on the needle guide system to a reference point. The reference point can be the same reference point used to determine the ideal angle. The current angle can be based on data provided from an accelerometer and a gyroscope of the needle guide system. This difference can be determined and updated at periodic intervals. The rate of this difference determination can be fast enough to be effectively real time.

In step 750, a difference between the ideal depth and a current depth of the needle on the needle guide system can be determined. The current depth can be calculated as a depth between a distal tip of the needle on the needle guide system to a reference point. The reference point can be the entry point, the target position, or a point located on the needle guide system. The current depth can be based on data provided by a sensor of the needle guide system. This difference can be determined and updated at periodic intervals. The rate of this difference determination can be fast enough to be effectively real time.

In some embodiments, the difference between the ideal depth and a current depth of the needle of the needle guide system can be determined visually by a clinician. Such visual indications can be provided as part of the needle guide system such as a graduated scale, mechanical stop, color coding, or by any other suitable visual mechanism.

In step S760, an icon can be displayed on the needle guide system that indicates the relative position of the ideal angle to the current angle, as previously described.

In step S770, an icon can be displayed on the needle guide system that indicates the relative position of the ideal depth to the current depth, as previously described.

The above-described embodiments of the present disclosure can be implemented in any of numerous ways. For example, the embodiments can be implemented using hardware, software, or a combination thereof. When implemented in software, the software code can be executed on any suitable computer, processor, or collection of processors, and circuitry whether provided in a single computer or distributed among multiple computers. Such processors can be implemented as integrated circuits, with one or more processors in an integrated circuit component. Though, a processor can be implemented using circuitry in any suitable format.

Additionally, or alternatively, the above-described embodiments can be implemented as a non-transitory computer readable storage medium embodied thereon a program executable by a processor that performs a method of various embodiments.

Also, the various methods or processes outlined herein can be coded as software that is executable on one or more processors that employ any one of a variety of operating systems or platforms. Additionally, such software can be written using any of a number of suitable programming languages and/or programming or scripting tools, and also can be compiled as executable machine language code or intermediate code that is executed on a framework or virtual machine. Typically, the functionality of the program modules can be combined or distributed as desired in various embodiments.

Also, the embodiments of the present disclosure can be embodied as a method, of which an example has been provided. The acts performed as part of the method can be ordered in any suitable way. Accordingly, embodiments can be constructed in which acts are performed in an order different than illustrated, which can include performing some acts concurrently, even though shown as sequential acts in illustrative embodiments.

It should be understood that the foregoing description is only illustrative of the present invention. Various alternatives and modifications can be devised by those skilled in the art without departing from the present invention. Accordingly, the present invention is intended to embrace all such alternatives, modifications, and variances that fall within the scope of the appended claims.

## Claims

1. A needle guide device for a needle, comprising:
a controller; and
a display driven by the controller and configured to indicate a difference between an ideal angle and a current angle of the needle.

2. The device of claim 1, including the needle and, optionally, wherein the needle is replaceable.

3. The device of claim 1 or 2, wherein the display indicates the current angle and, optionally, wherein the current angle is based on data from a gyroscope of the needle guide device.

4. The device of claim 1, 2 or 3, wherein the difference between the ideal angle and the current angle is based on data from a gyroscope and an accelerometer of the needle guide device.

5. The device of any one of claims 1 to 4, wherein:
the display is further configured to indicate a distance between a current position of the needle and a target position, and, optionally, wherein the distance between the current position of the needle and the target position is based on data from a distance sensor of the needle guide device; and/or
the device comprises a distance measurer to determine a distance between a current position of the needle and a target position.

6. The device of any one of claims 1 to 5, wherein the display indicates the current angle as a reticle and the ideal angle as an icon.

7. A method of guiding a needle comprising:
determining an ideal angle from an entry point of a needle of needle guide in a patient to a target position of the needle; and
displaying an icon on the needle guide that indicates a difference between a current angle of the needle guide and the ideal angle.

8. The method of claim 7, further comprising determining a difference between an ideal depth to the target position and the current depth of the needle.

9. The method of claim 7 or 8, wherein the icon indicates a difference between a current depth of the needle and an ideal depth of the needle to the target position, and, optionally, wherein the icon changes size to indicate the difference between the current depth and the ideal depth.

10. The method of claim 7, 8 or 9, wherein:
the current angle is determined based on data from a gyroscope and an accelerometer of the needle guide; and/or
wherein the current angle is displayed as a reticle.

11. A needle guide system comprising:
the needle guide according to any one of claims 1 to 6; and
a computer in communication with the needle guide and capable of determining the ideal angle and transmitting the ideal angle to the needle guide.

12. The system of claim 11, wherein the computer is further capable of determining an ideal depth of a needle of the needle guide between an entry point of a needle of the needle guide in a patient to a target position of the needle for treatment of the patient.

13. The system of claim 11 or 12, further comprising a distance measurer to determine a distance between a current position of the needle and a target position of the needle for treatment of the patient.

14. A non-transitory computer-readable medium including executable instructions that when executed by a processor cause the processor to perform the steps of:
determining a difference between an ideal angle that is an angle from an entry point of a needle of a needle guide in a patient to a target position of the needle for treatment of the patient; and
driving a display to display an icon on the needle guide that indicates a difference between a current angle of the needle guide and the ideal angle.

15. The non-transitory computer-readable medium of claim 14, further causing the processor to drive the display to display the icon to indicate a difference between a current depth of the needle in the patient and an ideal depth of the needle to the target position.
